(19) Europäisches Patentamt / European Patent Office / Office européen des brevets

(11) EP 1 987 879 A1

(12) **EUROPEAN PATENT APPLICATION**
published in accordance with Art. 158(3) EPC

(43) Date of publication:
**05.11.2008 Bulletin 2008/45**

(21) Application number: **07714413.7**

(22) Date of filing: **16.02.2007**

(51) Int Cl.:
***B01J 31/28*** *(2006.01)* ***B01J 37/03*** *(2006.01)*
***C07C 29/149*** *(2006.01)* ***C07C 29/154*** *(2006.01)*
***C07C 31/04*** *(2006.01)* ***C07B 61/00*** *(2006.01)*

(86) International application number:
**PCT/JP2007/052883**

(87) International publication number:
**WO 2007/094468 (23.08.2007 Gazette 2007/34)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **17.02.2006 JP 2006041575**

(71) Applicant: **Nippon Steel Engineering Co., Ltd**
**Tokyo 100-8071 (JP)**

(72) Inventors:
- **FUJIMOTO, Kenichiro,**
  **c/o NIPPON STEEL CORPORATION**
  **Futtsu-shi, Chiba 293-8511 (JP)**
- **YAMANE, Noriyuki,**
  **c/o NIPPON STEEL CORPORATION**
  **Futtsu-shi, Chiba 293-8511 (JP)**

(74) Representative: **Vossius & Partner**
**Siebertstrasse 4**
**81675 München (DE)**

# (54) CATALYST FOR METHANOL SYNTHESIS AND PRODUCTION METHOD THEREOF, AND METHOD FOR PRODUCING METHANOL

(57) The present invention relates to a catalyst for methanol synthesis, in which methanol is synthesized via a formic ester and the reaction is carried out under the presence of a starting material gas, which contains hydrogen and at least one of carbon monoxide and carbon dioxide, and an alcohol as a solvent, wherein the catalyst includes Cu, Mg, Na, and an alkali metal formate salt.

FIG. 1

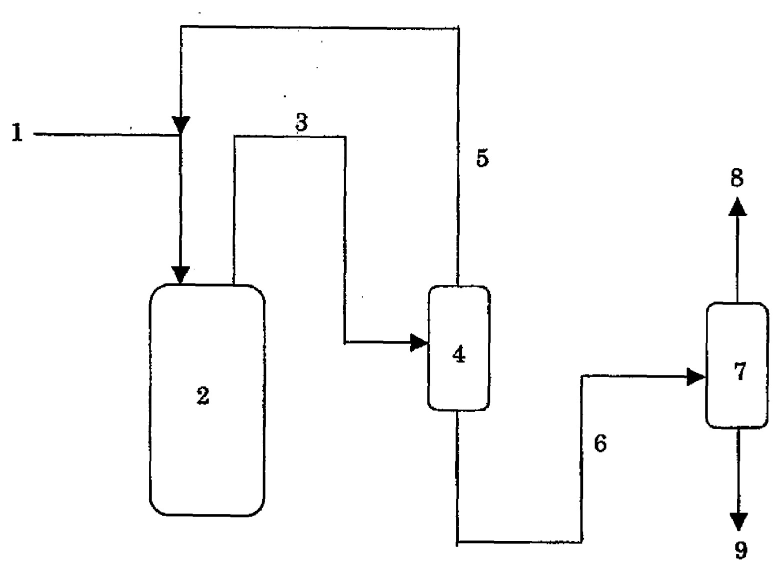




Printed by Jouve, 75001 PARIS (FR)

**Description**

TECHNICAL FIELD

The present invention relates to a catalyst for methanol synthesis, a method for producing the catalyst, and a method for producing methanol. More specifically, the present invention relates to a catalyst which is highly active when producing methanol from hydrogen and a carbon source, i.e., either carbon monoxide or carbon dioxide, and a method for obtaining a product with high efficiency using this catalyst.
Priority is claimed on Japanese Patent Application No. 2006-041575, filed February 17, 2006, the contents of which are incorporated herein by reference.

BACKGROUND ART

Generally, in the industrial synthesis of methanol, carbon monoxide and hydrogen (synthesis gas) obtained by steam reforming of a natural gas mainly composed of methane are used as the starting materials and the synthesis is carried out by a fixed-bed gas phase process using a copper/zinc-based catalyst or the like under severe conditions of a temperature of 200 to 300°C and a pressure of 5 to 25 MPa (Non-patent Document 1). A generally accepted explanation for the reaction mechanism is that it is a successive reaction where methanol and water are first produced due to the hydrogenation of carbon dioxide and then the produced water reacts with carbon monoxide to produce carbon dioxide and hydrogen (water-gas shift reaction).

[Chemical Formula 1]

$$CO_2 + 3H_2 \rightarrow CH_3OH + H_2O \qquad (1)$$

$$H_2O + CO \rightarrow CO_2 + H_2 \qquad (2)$$

$$CO + 2H_2 \rightarrow CH_3OH \qquad (3)$$

Although this reaction is an exothermic reaction, efficient heat extraction is difficult to achieve because of poor thermal conductivity in the gas phase method, and a process of lowering the one-pass conversion and recycling unreacted high-pressure starting material gas is employed, which has, however, a severe problem in the efficiency. Despite the above problem, the fixed-bed gas phase method is not easily prone to reaction inhibition by water or carbon dioxide contained in the synthesis gas and various plants are now operated by making use of this advantageous property.
On the other hand, various methods of synthesizing methanol in a liquid phase and thereby increasing the heat extraction rate have been studied. Among them, a method of using a catalyst having high activity at low temperatures (approximately from 100 to 180°C) is also thermodynamically advantageous for the production system, and thus drawing attention (Non-patent Document 2 and the like). However, it has been reported that water and carbon dioxide, which are always contained in the synthesis gas, decrease the catalytic activity in these methods, and thus none of them have been put into practice, although they use an alkali metal alkoxide as a catalyst (Non-patent Document 3). This is because the highly active alkali metal alkoxide changes into a low active, stable formate salt or the like during the reaction. In order to prevent the decrease of the catalytic activity, water and carbon dioxide in the starting material gas need to be removed down to the order of ppb. However, this is not realistic, since the production cost increases if such a pretreatment is carried out
To date, the present inventors have discovered the following system (Patent Document 1), in which, as the catalyst whose catalytic activity is little decreased due to water and carbon dioxide, one or both of a catalyst based on an alkali metal (except alkali metal alkoxide) and an alkaline earth metal-based catalyst are used under the presence of a hydrogenolysis catalyst. The above Patent Document 1 describes Cu/Mn, Cu/Re, and the like as effective hydrogenolysis catalysts. Further, the present inventors discovered that Cu/MgO has a particularly high activity in their later studies, but it is also turned out that the decrease of the catalytic activity over time is observed in the case of using Cu/MgO.
[Patent Document 1] Japanese Unexamined Patent Application, First Publication No. 2001-862701
[Non-patent Document 1] J. C. J. Bart et al., Catal. Today, 2,1 (1987)
[Non-patent Document 2] Seiichi Ohyama, Petrotech, 18(1), 27 (1995)
[Non-patent Document 3] S. Ohyama, Applied Catalysis A: General, 180, 217 (1999)

## DISCLOSURE OF INVENTION

## PROBLEMS TO BE SOLVED BY THE INVENTION

An object of the present invention is to solve the above problems and to provide a catalyst whose catalytic activity decreases only slightly even when a small amount of carbon dioxide, water, and the like are present in the starting material gas for methanol synthesis and which is also capable of synthesizing a formic ester and methanol under low temperature and pressure conditions, a method for producing the catalyst, and a method for synthesizing methanol in the liquid phase using the catalyst.

## MEANS FOR SOLVING THE PROBLEMS

The present invention is characterized by the following aspects.
Aspect (1) is a catalyst for methanol synthesis, where methanol is synthesized via a formic ester, which carries out a reaction under the presence of a starting material gas containing hydrogen and at least one of carbon monoxide and carbon dioxide, and an alcohol as a solvent, the catalyst including Cu, Mg, Na, and an alkali metal formate salt.
Aspect (2) is the catalyst for methanol synthesis according to aspect (1) in which the alkali metal formate salt is potassium formate.
Aspect (3) is a catalyst for methanol synthesis, where methanol is synthesized via a formate ester, which carries out a reaction under the presence of a source gas containing hydrogen and at least one of carbon monoxide and carbon dioxide, and an alcohol as a solvent, the catalyst for methanol synthesis containing Cu, Mg, Na, and an alkali metal-based catalyst which can change into an alkali metal formate salt.
Aspect (4) is the catalyst for methanol synthesis according to aspect (3) in which the alkali metal-based catalyst which can change into an alkali metal formate salt is an alkali metal carbonate salt.
Aspect (5) is the catalyst for methanol synthesis according to any one of aspects (1) to (4), wherein said Na is loaded as a carbonate salt or a formate salt on a Cu/MgO solid catalyst.
Aspect (6) is a method for producing the catalyst for methanol synthesis according to aspect (5), including the steps of preparing a Cu/MgO solid catalyst, and loading Na on the solid catalyst.
Aspect (7) is a method for producing the catalyst for methanol synthesis according to aspect (5), including the steps of preparing Cu/MgO by coprecipitation method, and loading said Na on said Cu/MgO by impregnation method.
Aspect (8) is a method for producing the catalyst for methanol synthesis according to aspect (5), including the step of preparing said Cu/MgO by coprecipitation method while maintaining a constant pH within a range of 8 to 11.
Aspect (9) is a method for producing methanol, including the steps of reacting a starting material gas containing hydrogen and at least one of carbon monoxide and carbon dioxide under the presence of an alcohol, an alkali metal formate salt, and a solid catalyst containing Cu, Mg, and Na, thereby producing a formic ester and methanol; and hydrogenating the formic ester thus obtained to produce methanol.
Aspect (10) is a method for producing methanol including the steps of reacting a starting material gas containing hydrogen and at least one of carbon monoxide and carbon dioxide under the presence of an alcohol, an alkali metal formate salt, and a solid catalyst containing Cu, Mg, and Na; separating products obtained from a reaction system; and hydrogenating the formic ester in the products by using a hydrogenolysis catalyst to produce methanol.
Aspect (11) is the method for producing methanol according to aspect (9) or (10), wherein an alkali metal-based catalyst which can change into an alkali metal formate salt during reaction is used instead of the alkali metal formate salt
Aspect (12) is the method for producing methanol according to any one of aspects (9) to (11), wherein the alkali metal formate salt is potassium formate.
Aspect (13) is the method for producing methanol according to aspect (12), wherein the alkali metal-based catalyst which can change into an alkali metal formate salt during reaction is an alkali metal carbonate salt.
Aspect (14) is the method for producing methanol according to any one of aspects (9) to (13), wherein the alcohol is a primary alcohol.

## EFFECTS OF THE INVENTION

When methanol is produced in a system of the present invention where a catalyst containing Cu, Mg, and Na

coexists with an alkali metal formate salt under the presence of a synthesized starting material gas, which contains hydrogen and at least one of carbon monoxide and carbon dioxide, and a solvent alcohol, it is possible to synthesize methanol stably at high efficiency in a continuous reaction under low temperature and pressure conditions. Moreover, it is possible to produce methanol at low cost since the extent of decrease of the catalytic activity remains low even when a small amount of water, carbon dioxide, or the like is mixed in the synthesized starting material gas.

BRIEF DESCRIPTION OF THE DRAWING

FIG 1 shows a reactor for conducting liquid phase synthesis of methanol at low temperatures according to the present invention.

FIG. 2 shows a temporal change in CO conversion in the reaction described in Example 1.

FIG 3 shows a temporal change in CO conversion in the reaction described in Comparative Example 1.

FIG 4 shows a temporal change in CO conversion in the reaction described in Comparative Example 2.

DESCRIPTION OF THE REFERENCE SYMBOLS

1 Synthesis gas
2 Semi-batch reactor
3 Mixture of product and unreacted gas
4 Cooler
5 Unreacted gas
6 Liquid mixture of formic ester and methanol
7 Distillation column
8 Formic ester
9 Methanol

BEST MODE FOR CARRYING OUT THE INVENTION

The present invention will be described in detail below.

As a result of intensive studies, the present inventors discovered the following to complete the present invention. That is, in the continuous reaction using a semi-batch system where a catalyst and a solvent are fed to a reactor and a starting material gas is also supplied thereto, it is possible to produce a high yield of methanol from at least one of carbon monoxide and carbon dioxide, hydrogen, and an alcohol, when a catalyst containing Cu, Mg, and Na is used in addition to an alkali metal formate salt.

For example, methanol can be produced continuously by the reaction process shown in FIG. 1. In addition to an alkali metal formate salt, a solid catalyst containing Cu, Mg, and Na is fed with a solvent alcohol to a semi-batch reactor 2, and then synthesis gas 1 is supplied thereto. A mixture 3 of products (i.e., formic ester and methanol) and unreacted gas collected at the reactor outlet is cooled in a cooler 4 to be separated into unreacted gas 5 and a liquid mixture 6 of a formic ester and an alcohol. The latter mixture is further separated into a formic ester 8 and methanol 9 in a distillation column 7 provided in the next step. When the conversion is low, it is possible to supply the unreacted gas again to the semi-batch reactor 2. However, when the product is obtained at a high yield, the unreacted gas is used as a heat source (fuel) in the production of synthesis gas.

Examples of the alkali metal formate salt include potassium formate, sodium formate, cesium formate, and rubidium formate. Potassium formate is particularly preferable since its use enhances catalytic activity.

In addition, it is also possible to use an alkali metal-based catalyst, which can adopt the form of a formate salt during the reaction, instead of the alkali metal formate salt, and the form thereof at the time of being fed to the reaction is not particularly limited.

Examples of such alkali metal-based catalysts include potassium carbonate and potassium methoxide. When potassium carbonate is used, it is postulated that potassium carbonate changes into potassium formate by the following reaction. Even when the alkali metal-based catalyst is fed in other forms, it is assumed that the catalyst changes into a formate salt, which is more stable.

[Chemical Formula 2]

$$K_2CO_3 + H_2O \rightarrow 2KOH + CO_2 \quad (4)$$

$$KOH + CO \rightarrow HCOOK \quad (5)$$

Specifically, the solid catalyst which coexists with the abovementioned alkali metal formate salt or the alkali metal-based catalyst capable of changing into an alkali metal formate salt is Cu/$MgO_x$/Na (wherein, X is a chemically allowable value) and examples thereof include Cu/$MgO_x$/HCOONa (wherein, X is a chemically allowable value). The method for preparing Cu/$MgO_x$ is not particularly limited and an ordinary method such as impregnation method, precipitation method, sol-gel method, coprecipitation method, ion exchange method, kneading method and drying method may be used, although good results are readily obtained by the use of a coprecipitation method. The CO conversion varies considerably depending on the pH, which is maintained constant when preparing a catalyst with coprecipitation method. The pH when preparing the Cu/$MgO_x$ catalyst is preferably 8 to 11, more preferably 8.5 to 10.5, and even more preferably 9 to 10. The pH range exceeding 11 is not economical since the amount of an alkaline compound used as a precipitant in order to maintain a highly alkaline atmosphere increases distinctively. The method for loading an Na salt to Cu/$MgO_x$ is not particularly limited and the abovementioned ordinary methods may be used, although good results are readily obtained by the use of impregnation method or drying method. The loading amount of Na relative to Cu/$MgO_x$ is not particularly limited as long as it is at least the minimum amount where its effects are exhibited. However, the amount is preferably within a range of 0.1 to 60 mass%, more preferably 1 to 40 mass%, and even more preferably 3 to 30 mass%. In addition, the loaded Na salt is preferably sodium formate, sodium carbonate, or the like. Cu/$MgO_x$ which does not load Na salts has high activity, but the decrease of the catalytic activity over time is also observed in a continuous reaction in the case of using Cu/$MgO_x$ which does not load Na salts. On the other hand, by loading these Na salts, the decrease of the catalytic activity over time, which is a problem in the case of using Cu/$MgO_x$, can be suppressed. Moreover, load of Na salts slightly increases the catalytic activity. Accordingly, it can be said that the addition of the alkali metal carbonate salt is effective for improving catalytic activity and for suppressing the decrease of catalytic activity.

The abovementioned solid catalyst containing Cu, Mg, and Na exhibits a catalytic action mainly in the hydrogenolysis of the produced formic esters, although a catalytic action thereof is also exhibited in the reaction for inserting CO in a solvent alcohol.

The alcohol used in the reaction may be an alcohol where a hydroxyl group is bonded with a chained or an alicyclic hydrocarbon, phenol or a substitution product thereof, or thiol or a substitution product thereof. Although these alcohols may be any of primary, secondary and tertiary alcohols, primary alcohols are preferable due to their reaction efficiency. Lower alcohols such as methyl alcohols and ethyl alcohols are most commonly used.

Although the reaction can be performed in either the liquid phase or the gas phase, a system where moderate conditions can be selected may be employed. Specifically, preferable conditions are the temperature of 70 to 250°C and the pressure of 3 to 100 atmospheres, more preferably the temperature of 120 to 200°C and the pressure of 15 to 80 atmospheres, although conditions are not limited to the above. Although the amount of alcohol used is not limited as long as it is sufficient enough for the reaction to proceed, an even larger amount of alcohol may be used as a solvent In addition, an organic solvent other than alcohols may be used appropriately in combination during the above reaction.

The obtained formic ester may be purified by an ordinary method such as distillation, but may also be used as it is in the production of methanol. In other words, methanol can be produced by hydrogenating the formic ester.

A hydrogenolysis catalyst is used in the hydrogenolysis process. For example, a common hydrogenolysis catalyst which is based on Cu, Pt, Ni, Co, Ru, and Pd may be employed, but the Cu/$MgO_x$/Na of the present invention can also be used. By making these common hydrogenolysis catalysts coexist in the aforementioned reaction system where a formic ester and methanol are produced from the starting material gas and an alcohol, the selectivity for methanol increases, and thus methanol can be produced efficiently.

In addition, when it is difficult to produce methanol in the one-step process under a reaction condition where the selectivity for formic esters is high, it is also possible to obtain methanol by first separating the products obtained in the reaction from the reaction system using a distillation method or the like and then hydrogenating the formic ester in the products in the presence of a hydrogenolysis catalyst and hydrogen.

Although methanol can be obtained with the method using the catalyst of the present invention even if $CO_2$ is the only carbon source in the starting material gas, the catalytic activity is lower compared to that if CO is the sole carbon source in the starting material gas. In addition, the lower the concentrations of $CO_2$ and $H_2O$ in the starting material gas having CO as the main carbon source, the higher the yield of methanol. However, the CO conversion and the

methanol yield are hardly affected even when the starting material gas contains about 1% each of $CO_2$ and $H_2O$. However, when the starting material gas contains $CO_2$ and $H_2O$ at a concentration higher than the above concentration, the CO conversion and the methanol yield decrease.

The process for producing formic esters and methanol according to the present invention is presumably based on the following reaction (a case where the alcohol used in the reaction is an alcohol in which a hydroxyl group is bonded with a chained or an alicyclic hydrocarbon is shown as an example).

[Chemical Formula 3]

$$ROH + CO \rightarrow HCOOR \quad (6)$$

$$HCOOR + 2H_2 \rightarrow CH_3OH + ROH \quad (7)$$

(wherein, R represents an alkyl group)

Accordingly, the starting materials for producing methanol are at least one of the following combinations of compounds; i.e., carbon monoxide and hydrogen, and carbon dioxide and hydrogen. Alcohols can be recovered and reused. According to the present invention, decrease of the catalytic activity is small even if a small amount of water or carbon dioxide is present in the starting material gas.

EXAMPLES

The present invention will be described in further detail using Examples. However, the present invention is not limited to these Examples.

CO conversion described below in Examples and Comparative Examples are calculated by the following formula.

$$\text{CO conversion (\%)} = [1-(\text{number of moles of CO collected after the reaction})/(\text{number of moles of CO fed in the reaction})] \times 100$$

[Example 1]

In an autoclave having a content volume of 100 ml, 10 mmol of potassium formate, 2 mmol of calcium formate as well as 3 g of a $Cu/MgO_x/Na$, in which 9.0 mass% of $Na_2CO_3$ relative to $Cu/MgO_x$ that was prepared from Cu $(NO_3)_2 \cdot 3H_2O$ and $Mg(NO_3)_2 \cdot 6H_2O$ as starting materials by coprecipitation method while maintaining the pH at 10.0 was impregnated therein, and consequently loaded thereon, was added to 27.8 ml of methanol as a solvent, and then synthesis gas (i.e., 30.00 vol% of CO, 60.0 vol% of hydrogen, and Ar as balance) was provided thereto at the rate of 60 ml/min to cany out a reaction at 160°C under a pressure of 3.5 MPa. The reaction products were analyzed by gas chromatography. The temporal change in CO conversion is shown in FIG. 2. As compared with the case of using a $Cu/MgO_x$ which did not load $Na_2CO_3$, described later in Comparative Example 1, the case of using $Cu/MgO_x/Na_2CO_3$ above resulted in the stable CO conversion.

[Comparative Example 1]

The reaction was performed by the method described in Example 1 except that a $Cu/MgO_x$ which did not load $Na_2CO_3$ was added instead of the $Cu/MgO_x/Na_2CO_3$ as the hydrogenolysis catalyst. The temporal change in CO conversion is shown in FIG. 3. FIG. 3 shows that the CO conversion tends to decrease as time progresses.

[Comparative Example 2]

The reaction was performed by the method described in Examples 1 except that a $Cu/MnO_x$ which did not load $Na_2\ CO_3$ was added instead of the $Cu/MgO_x/Na_2CO_3$ as the hydrogenolysis catalyst. The temporal change in CO

conversion is shown in FIG. 4. FIG. 4 shows that the CO conversion is low as compared with that in the case of using Cu/MgOx-based catalysts, and tends to decrease as time progresses.

As is clear from the above Example and Comparative Examples, Cu/$MgO_x$ has high activity as compared with Cu/$MnO_x$, but the activity decrease as time progresses. On the other hand, in the case of using Cu/$Mgo_x$/$Na_2CO_3$ which loads $Na_2CO_3$ to Cu/$MgO_x$, the activity does not decrease. Therefore, the use of the catalyst enables the cost for the catalyst to be decreased, and enables methanol to be produced inexpensively.

INDUSTRIAL APPLICABILITY

The present invention relates to a catalyst for methanol synthesis, in which methanol is synthesized via a formic ester and the reaction is carried out under the presence of a starting material gas, which contains hydrogen and at least one of carboy monoxide and carbon dioxide, and an alcohol as a solvent, wherein the catalyst includes, in addition to an alkali metal formate salt, a catalyst containing Cu, Mg, and Na. According to the catalyst for methanol synthesis of the present invention, it is possible to synthesize methanol stably at a high efficiency in a continuous reaction under a low temperature and low pressure conditions. Moreover, it is possible to produce methanol at a low cost since the extent of decrease of the catalytic activity remains low even when a small amount of water, carbon dioxide, or the like is mixed in the synthesized starting material gas.

## Claims

1. A catalyst for methanol synthesis, where methanol is synthesized via a formic ester, which carries out a reaction under the presence of a starting material gas containing hydrogen and at least one of carbon monoxide and carbon dioxide, and an alcohol as a solvent,
the catalyst comprising: Cu, Mg, Na, and an alkali metal formate salt.

2. The catalyst for methanol synthesis according to Claim 1, wherein the alkali metal formate salt is potassium formate.

3. A catalyst for methanol synthesis, where methanol is synthesized via a formic ester, which carries out a reaction under the presence of a starting material gas containing hydrogen and at least one of carbon monoxide and carbon dioxide, and an alcohol as a solvent,
the catalyst comprising: Cu, Mg, Na, and an alkali metal-based catalyst which can change into an alkali metal formate salt.

4. The catalyst for methanol synthesis according to Claim 3, wherein the alkali metal-based catalyst which can change into an alkali metal formate salt is an alkali metal carbonate salt.

5. The catalyst for methanol synthesis according to any one of Claims 1 to 4, wherein Na is loaded as a carbonate salt or a formate salt on a Cu/MgO solid catalyst.

6. A method for producing the catalyst for methanol synthesis described in Claim 5, comprising the steps of:

   preparing a Cu/MgO solid catalyst; and
   loading Na on the solid catalyst.

7. A method for producing the catalyst for methanol synthesis described in Claim 5, comprising the steps of:

   preparing said Cu/MgO by coprecipitation method; and
   loading said Na on the Cu/MgO by impregnation method.

8. A method for producing a catalyst for methanol synthesis described in Claim 5, the method comprising:

   preparing said Cu/MgO by coprecipitation method while maintaining a constant pH within a range of 8 to 11.

9. A method for producing methanol, comprising:

   reacting a starting material gas containing hydrogen and at least one of carbon monoxide and carbon dioxide under the presence of an alcohol, an alkali metal formate salt, and a solid catalyst containing Cu, Mg, and Na,

thereby producing a formic ester and methanol; and
hydrogenating the formic ester thus obtained to produce methanol.

**10.** A method for producing methanol comprising:

reacting a starting material gas containing hydrogen and at least one of carbon monoxide and carbon dioxide under the presence of an alcohol, an alkali metal formate salt, and a solid catalyst containing Cu, Mg, and Na;
separating products obtained from a reaction system; and
hydrogenating the formic ester in the products by using a hydrogenolysis catalyst to produce methanol.

**11.** The method for producing methanol according to Claim 9 or 10, wherein an alkali metal-based catalyst which can change into an alkali metal formate salt during reaction is used instead of the alkali metal formate salt.

**12.** The method for producing methanol according to any one of Claims 9 to 11, wherein the alkali metal formate salt is potassium formate.

**13.** The method for producing methanol according to Claim 12, wherein the alkali metal-based catalyst which can change into an alkali metal formate salt during reaction is an alkali metal carbonate salt.

**14.** The method for producing methanol according to any one of Claims 9 to 13, wherein the alcohol is a primary alcohol.

FIG. 1

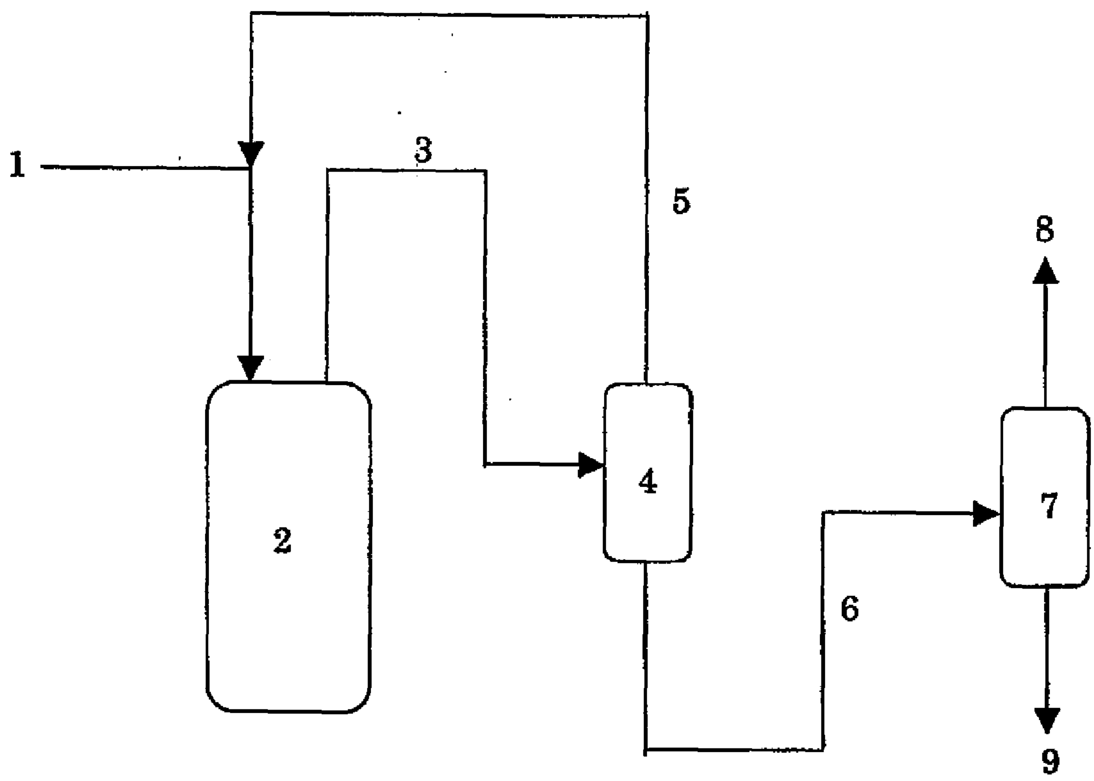

FIG. 2

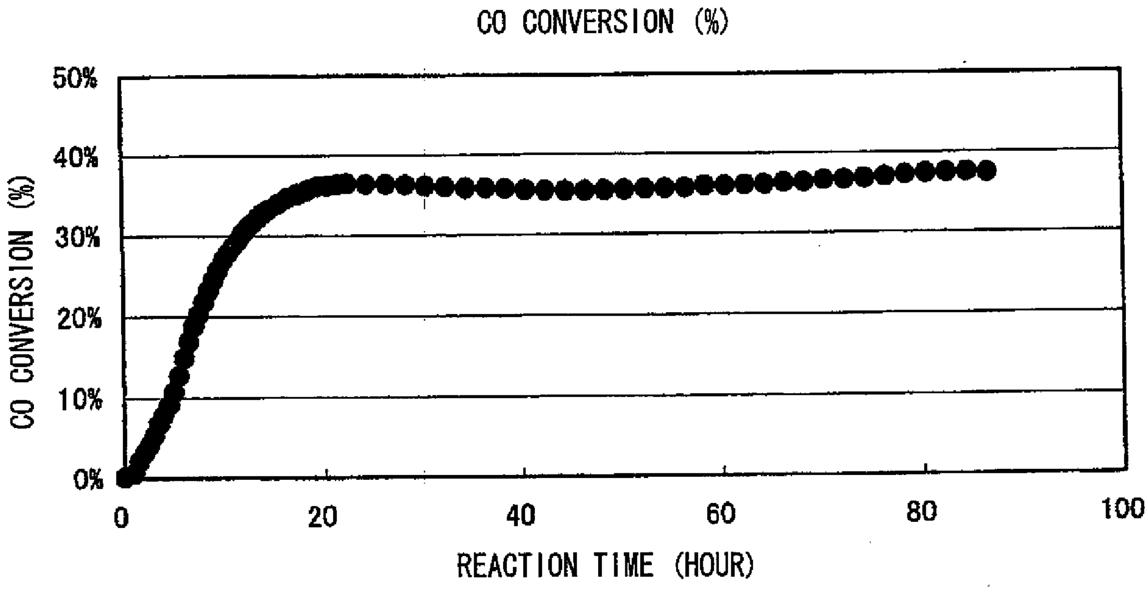
CO CONVERSION (%)
50%
40%
30%
20%
10%
0%
CO CONVERSION (%)
0
20
40
60
80
100
REACTION TIME (HOUR)

FIG. 3

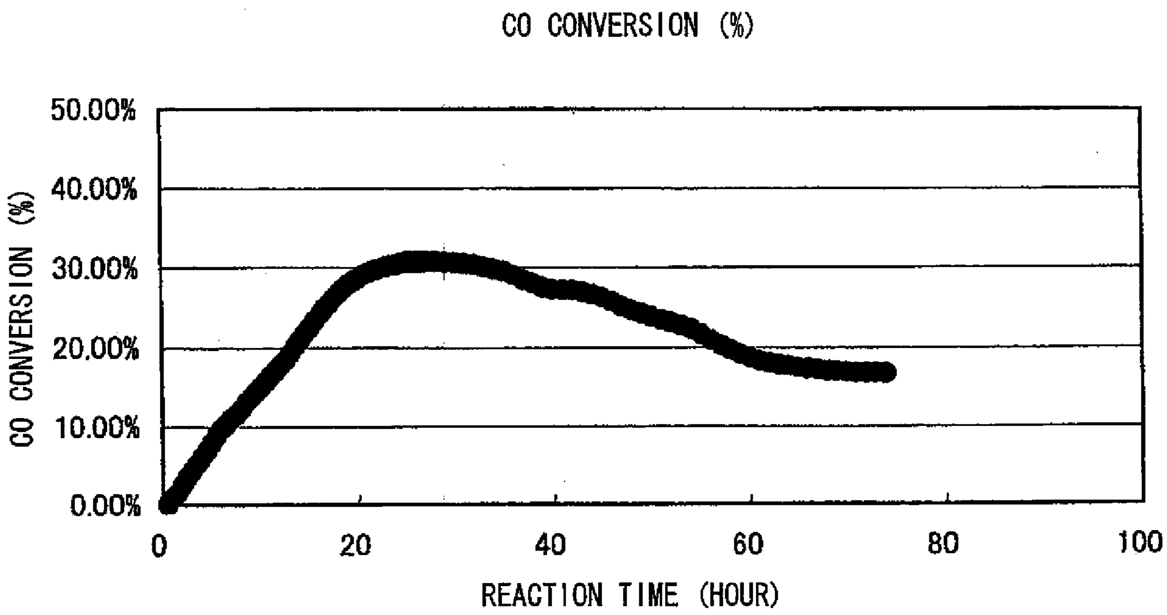
CO CONVERSION (%)
50.00%
40.00%
30.00%
20.00%
10.00%
0.00%
CO CONVERSION (%)
0
20
40
60
80
100
REACTION TIME (HOUR)

FIG. 4

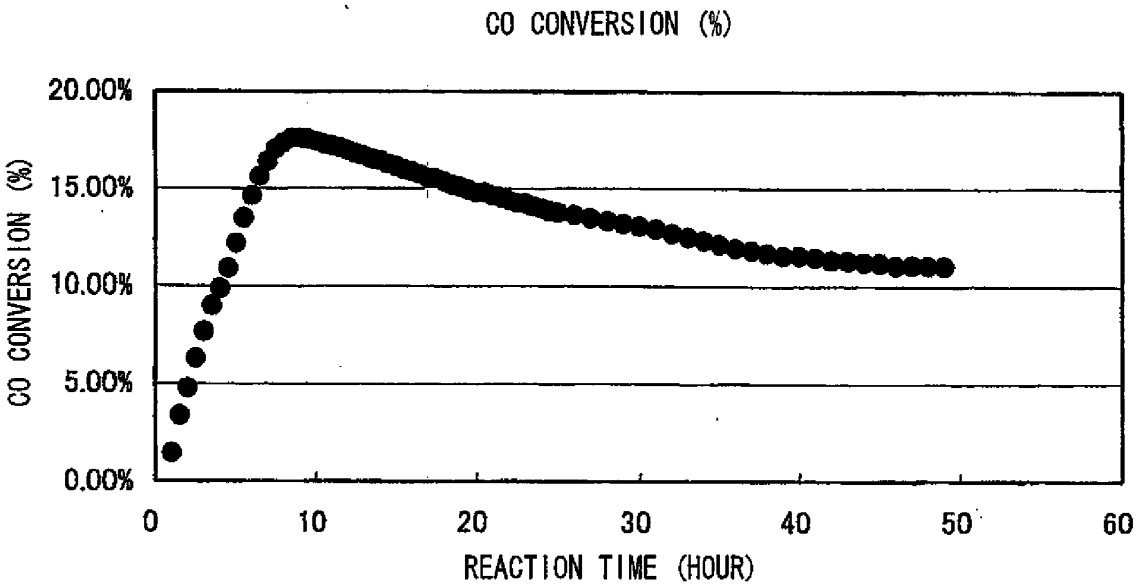
CO CONVERSION (%)
20.00%
15.00%
10.00%
5.00%
0.00%
CO CONVERSION (%)
0
10
20
30
40
50
60
REACTION TIME (HOUR)

**INTERNATIONAL SEARCH REPORT**

International application No.
PCT/JP2007/052883

A. CLASSIFICATION OF SUBJECT MATTER
***B01J31/28***(2006.01)i, ***B01J37/03***(2006.01)i, ***C07C29/149***(2006.01)i, ***C07C29/154***(2006.01)i, ***C07C31/04***(2006.01)i, ***C07B61/00***(2006.01)n

According to International Patent Classification (IPC) or to both national classification and IPC

B. FIELDS SEARCHED

Minimum documentation searched (classification system followed by classification symbols)
B01J31/28, B01J37/03, C07C29/149, C07C29/154, C07C31/04, C07B61/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2007 |
| Kokai Jitsuyo Shinan Koho | 1971-2007 | Toroku Jitsuyo Shinan Koho | 1994-2007 |

Electronic data base consulted during the international search (name of data base and, where practicable, search terms used)
JST7580(JDream2), JSTPlus(JDream2)

C. DOCUMENTS CONSIDERED TO BE RELEVANT

| Category* | Citation of document, with indication, where appropriate, of the relevant passages | Relevant to claim No. |
|---|---|---|
| Y | JP 2005-095872 A (Nippon Steel Corp.), 14 April, 2005 (14.04.05), Claims 1 to 8; Par. Nos. [0017] to [0021] (Family: none) | 1-14 |
| Y | JP 2005-126427 A (Nippon Steel Corp.), 19 May, 2005 (19.05.05), Par. Nos. [0016], [0039] & WO 2005/030686 A1 | 1-14 |
| Y | Yasuyuki ARAKURA et al., "Formic acid esters o Keiyu suru Gosei Gas kara no Teion Methanol Gosei", Dai 85 Kai Shokubai Toronkai Toronkai A Yokoshu, 24 March, 2000 (24.03.00), page 74 | 3-8,11-14 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 25 April, 2007 (25.04.07) | 15 May, 2007 (15.05.07) |

| Name and mailing address of the ISA/ | Authorized officer |
|---|---|
| Japanese Patent Office | |
| Facsimile No. | Telephone No. |

Form PCT/ISA/210 (second sheet) (April 2005)

**INTERNATIONAL SEARCH REPORT**

International application No.
PCT/JP2007/052883

C (Continuation). DOCUMENTS CONSIDERED TO BE RELEVANT

| Category* | Citation of document, with indication, where appropriate, of the relevant passages | Relevant to claim No. |
|---|---|---|
| Y | JP 6-319999 A (Exxon Research & Engineering Co.), 22 November, 1994 (22.11.94), Par. Nos. [0003] to [0006], [0021], [0022], [0027], [0028] & US 5387570 A & US 5508246 A & EP 0623382 A1 | 8 |
| A | JP 55-085530 A (Institut Francais du Petrole), 27 June, 1980 (27.06.80), & US 4291126 A & GB 2037179 A & DE 2949952 A1 & FR 2444654 A1 & FR 2506299 A1 | 1-14 |
| A | WO 2001/062701 A1 (Nippon Steel Corp.), 30 August, 2001 (30.08.01), & EP 1180511 A1 & US 2003/0013930 A1 & US 2004/0171704 A1 & US 7081547 B2 | 1-14 |
| A | JP 9-087217 A (Director General of Basic Industries Bureau of Ministry of International Trade and Industry), 31 March, 1997 (31.03.97), (Family: none) | 1-14 |
| A | JP 9-509881 A (Imperial Chemical Industries PLC), 07 October, 1997 (07.10.97), & US 5928985 A & WO 1995/023644 A1 | 1-14 |
| A | JP 59-098024 A (Shinnenryoyu Kaihatsu Gijutsu Kenkyu Kumiai), 06 June, 1984 (06.06.84), & US 4582858 A & EP 110357 A2 | 1-14 |
| A | JP 56-095137 A (Union Carbide Corp.), 01 August, 1981 (01.08.81), & US 4289710 A & EP 0031244 A1 | 1-14 |
| A | JP 59-116238 A (Imperial Chemical Industries PLC), 05 July, 1984 (05.07.84), & US 5137924 A & EP 0117944 A2 | 1-14 |

Form PCT/ISA/210 (continuation of second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2006041575 A
- JP 2001862701 A


### Non-patent literature cited in the description


- **J. C. J. BART et al.** *Catal. Today,* 1987, vol. 2, 1
- *Seiichi Ohyama, Petrotech,* 1995, vol. 18 (1), 27
- **S. OHYAMA.** *Applied Catalysis A: General,* 1999, vol. 180, 217